(19) Europäisches Patentamt
European Patent Office
Office européen des brevets

(11) Publication number: **0 274 444 A2**

(12) **EUROPEAN PATENT APPLICATION**

(21) Application number: 88300119.0

(22) Date of filing: 08.01.88

(51) Int. Cl.4: **C 08 B 37/16**
A 61 K 9/18, A 61 K 31/375

(30) Priority: 09.01.87 US 1720

(43) Date of publication of application: 13.07.88 Bulletin 88/28

(84) Designated Contracting States: AT BE CH DE ES FR GB GR IT LI LU NL SE

(71) Applicant: **Bristol-Myers Company**
**345 Park Avenue**
**New York New York 10154 (US)**

(72) Inventor: **Markarian, Berand M.**
**160 Water Edge**
**Conger New York 10920 (US)**

**Cohen, George L.**
**40 Gregory Lane**
**Warren New Jersey (US)**

(74) Representative: **Baillie, Iain Cameron et al**
**c/o Ladas & Parry Isartorplatz 5**
**D-8000 München 2 (DE)**

(54) **Solution inbuprofen complexes, compositions and processes for preparing the same.**

(57) Ibuprofen - cyclodextrin complexes wherein the cyclodextrin is selected from the group consisting of α-cyclodextrin, methylated-β-cyclodextrins and γ-cyclodextrin; processes and compositions also being disclosed.

EP 0 274 444 A2

**Description**

SOLUTION IBUPROFEN COMPLEXES, COMPOSITIONS AND PROCESSES FOR PREPARING THE SAME

This invention relates to certain cyclodextrin-ibuprofen complexes and processes for their preparation. More particularly it concerns complexes of ibuprofen with cyclodextrins selected from the group consisting of α-cyclodextrin, methylated-β-cyclodextrins and γ-cyclodextrin. For the sake of convenience α-cyclodextrin and γ-cyclodextrin are sometimes referenced to herein by the shorthand designation α-CD, and γ-CD respectively. The expression H-β-CD is sometimes used to designate heptakis-(2,6-0-methyl)-β-cyclodextrin.

Fig. 1 of the attached drawings is a graph in which the solubility of the ibuprofen is plotted against the various combinations of mole ratios of H-β-CD and ibuprofen, the pH of the various combinations being given on the vertical axis to the right.

Fig. 2, is a graph similar to that of Fig. 1, except that the mole ratio of γ-CD and ibuprofen is plotted.

Fig. 3, is a graph similar to that of Fig. 1 except that mole ratio of α-CD and ibuprofen is plotted.

Fig. 4 is a graph of a differential scanning calorometric study; I being the endothermic curve for ibuprofen, II being the curve for heptakis (2,6-di-O-methyl)-β-cyclodextrin and IV being the curve for the inclusion complex of ibuprofen and heptakis (2,6-O-methyl-β-cyclodextrin) prepared in accordance with the process of Example 1.

Fig. 5 are infrared spectra of the inclusion complex prepared in accordance with the process of Example 1, the physical mixture of ibuprofen and heptakis (2,6-di-O-methyl)-β-cyclodextrin, the heptakis (2,6-di-O-methyl)-β-cyclodextrin alone and ibuprofen alone, the characteristic peaks of the respective materials being shown.

The therapeutic use of ibuprofen, particularly in ingestable liquid form is limited by both its very low solubility and its bitter taste. This low solubility also limits and tuse of ibuprofen in liquids intended for topical application. It has now been found that the solubility of ibuprofen can be dramatically enhanced by forming complexes of ibuprofen with cyclodextrins selected from the group consisting of α-CD, methylated-β-cyclodextrins (e.g. H-β-CD) and γ-CD. These complexes have the same uses for which ibuprofen is used e.g. as an analgesic, anti-inflammatory, etc. agent.

The , α and β-Cyclodextrins are a well known classes of compounds that are known to form inclusion compounds with certain compounds (see article by Wolfram Saenger, "Cyclodextrin Inclusion Compounds in Research and Industry" in Angew. Chem. Int. Ed. Engl. 19, 344-362 (1980). The α-, β- and γ-cyclodextrins are cyclic oligosaccharides containing six, seven and eight glucose units, respectively, in their rings. Some are said to form inclusion compounds with certain small molecules that fit into their cavity which measure 5-8 Å units.

A number of methylated-β-cyclodextrins are known in the prior art that are useful for the purposes of the present invention. By way of example, mention may be made of such compounds as heptakis (2,6-di-O-methyl-β-cyclodextrin and heptakis (2,3,6-tri-O-methyl)-β-cyclodextrin. However, exceptionally good and unexpected results were obtained with heptakis (2,6-di-O-methyl)-β-cyclodextrin as will be discussed in my detail below. This, accordingly, is the cyclodextrin of choice for practicing the present invention.

It has been reported in the prior art that the taste of ibuprofen may be masked by forming an inclusion compound with β-cyclodextrin. (See Japanese Patent 56-46837, April 28, 1981 and 55-92341, July 12, 1980.) Although the solubility of ibuprofen-β-CD complex may be increased by 12 fold, [DahNan, D. Chow and Adel H. Karara, International Journal of Pharmaceutics, Volume 28, pp. 95-101 (1986], it is still low compared to preferred complexes of this invention which limits its utility.

Uekama et al (J. Pharm. Sciences, Volume 74, No. 8, p. 841-845 (1985) have reported the preparation of inclusion complexes of flurbiprofen with heptakis (2,6-di-O-methyl)-β-cyclodextrin and heptakis (2,3,6-tri-O-methyl)-β-cyclodextrin and increasing the solubility of the flurbiprofen. However, this reference says nothing with respect to the formation of ibuprofen inclusion complexes. Moreover, structurally flurbiprofen and ibuprofen are quite different as an examination of the formulas below will show:

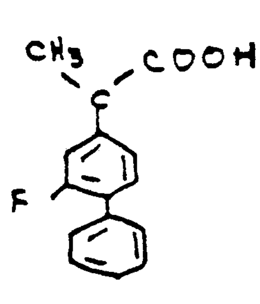

Flurbiprofen

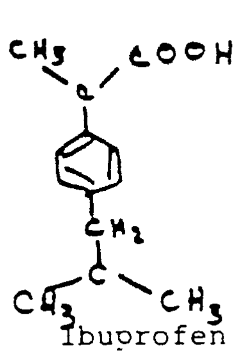

Ibuprofen

β-Cyclodextrin has also been reported as forming inclusion complexs with such drugs and flufenamic acid

and indomethacin. In this connection attention is invited to an article of N. Nambu et al., Chem. Pharm. Bull. Volume 26, No. 10. p. 2952 et seq. (1978). This, however, is not suggestive of the present invention.

The cyclodextrin-ibuprofens of the present invention are prepared by adding to a known amount of cyclodextrin dissolved in a solvent (e.g. water) an amount of ibuprofen in the desired mole ratios. The preferred mole ratios are as follows:

(a) about 9 moles of α-cyclodextrin to about 1 mole of ibuprofen;

(b) about 1 mole of methylated-β-cyclodextrin (e.g. heptakis 2,6-O-methyl-β-cyclodextrin) to about 1 mole of ibuprofen;

(c) about 1 mole of γ-cyclodextrin to about 2 moles of ibuprofen.

The mixture is mixed for an extended period of time at elevated temperatures (e.g., overnight at 55°C) cooled (e.g. to 25°C) and preferably filtered. The clear solution is rotavapped and the solid is collected.

The ibuprofen-cyclodextrin complexes of this invention are useful as analgesic/anti-inflammatory drugs and may be incorporated in any suitable dosage form. They may be formulated as ingestible liquids, (e.g. elixirs, syrups, suspensions) topical liquids, creams, ointments, lotions, suppositoreis etc. The complexes have been isolated as dry materials and in this form may be incorporated in such dosage forms as granulations, capsules, caplets, tablets, etc. The quantity of complex that will be contained in these dosage forms may vary depending on the particular dosage form selected. Usually, however, the daily average dose for the present complexes will be in the range of from 200 mg/day to about 9,000 mg/day with the preferred dose being from about 800 mg/day to about 6,000 mg/day.

The quantity of complex of this invention contained in each unit dose may also vary. These unit doses will be designed so that they may provide a therapeutically effect dose of the complex that can be conveniently taken by the user. As a general rule each unit dose will contain the present complexes in the range of from about 200 mg to about 3,000 mg. per unit dose with the preferred range being from about 400 mg. to about 1,500 mg. per unit dose.

As pointed above, a feature of the present invention is to convert ibuprofen into a form that has satisfactory water solubility characteristics by complexing the ibuprofen with certain cyclodextrins. The results in this regard range from say good, through excellent to extraodinary, depending upon the particular cyclodextrin selected for use. Thus when γ-cyclodextrin is used as the complexing agent (e.g. γ-cyclodextrin:ibuprofen mole ratio 1:2) there is about a 2-fold increase in ibuprofen water solubility. Although the solubility characteristics of the ibuprofen-γ-cyclodextrin complex are not as dramatic as those obtained with the other cyclodextrins there is nothing in the prior art which suggests the formation of such complexes. If α-cyclodextrin is chosen as the complexing agent (e.g. α-cyclodextrin:ibuprofen mole ratio of 9:1) there results an excellent 20-fold increase in water solubility . Where the heptakis(2,6-di-O-methyl)-β-cyclodextrin is selected (e.g. heptakis (2,6-di-O-methyl)-β-cyclodextrin:ibuprofen mole ratio 1:1) there is an extraordinary 180 fold increase in the water solubility of ibuprofen.

The following examples are given to further illustrate the present invention. It is to be understood, however, that the invention is not limited thereto.

Example 1

Preparation of heptakis(2,6-di-O-methyl)-β-cyclodextrin/ibuprofen complex

6.58g ($4.85 \times 10^{-3}$ moles) of heptakis (2,6-di-O-methyl-β)-cyclodextrin was dissolved in 100 ml water. To this solution 1g ($4.85 \times 10^{-3}$ moles) of ibuprofen was added. The solution was mixed by a magnetic stirrer. The solution was placed in a water bath at 50°C and stirred overnight.

After 24 hours, the solution was cooled at 25°C, kept for 2 hours, then transferred to 250 ml round bottom flask and rotavapped using Buchi model 110 rotavap equipment.

The solid, a white material was pulverized in a mortar.

Differential scanning calorometric studies (Figure 4) show that while ibuprofen alone (I) and cyclodextrin physical mixture show (III) show endothermic curves, the ibuprofen-cyclodextrin inclusion compound (IV) does not, implying a complete encapsulation of the ibuprofen molecule by the cyclodextrin cavity.

A shift of 16 $Cm^{-1}$ in the Fourier Transform Infrared Spectrum also indicate (Figure 5) that ibuprofen is entrapped by the cyclodextrin.

Example 2

Preparation of γ-cyclodextrin/ibuprofen complex

300 mg ($2.32 \times 10^{-4}$ moles) of γ-cyclodextrin was dissolved in 5 ml water. To this solution 97.4 ($4.72 \times 10^{-4}$ moles) of ibuprofen was added. The mixture was placed in a waterproof jar, placed in a water bath at 50°C and rotated for 24 hours. After 24 hours it was cooled to 25°C and kept there for 2 hours. The solution was filtered and the concentration of ibuprofen determined spectrophotemetrically. The solution was rotavapped and a solid obtained.

Example 3

Preparation of α-cyclodextrin/ibuprofen complex

613 mg ($6.34 \times 10^{-4}$ moles) of α-cyclodextrin was dissolved in distilled water. To this 14.3 mg ($6.93 \times 10^{-5}$ moles) of ibuprofen was added. The mixture was placed in a waterproof jar and this in turn in a water bath at 50°C. It was rotated overnight. After 24 hours the solution was cooled to 25°C, filtered and rotavapped. A white solid was obtained.

To measure the solubility of the complexes formed in accordance with the present invention and to determine the stoichiometry Job's Method of Continuous Variations was employed. (See P. Job, Ann. Chim. Volume 9, p. 113 (1928). See also Physical Pharmacy, A. Martin et al Chapt. 13; pp. 325 et seq, 1983).

Methods

For this method, combinations of ibuprofen with each cyclodextrin was prepared so that the total number of moles was constant but the mole ratio of the compounds varied from all cyclodextrin to all ibuprofen. The combinations were dispersed in 5 ml of distilled water, equilibrated for 24 hours at 50°C, cooled to room temperature, filtered through a 0.045μ filter and the concentration of ibuprofen was determined at 263 and 222 nm with a Hewlett-Packard 8450A Spectrophotometer. The ratio of ibuprofen to cyclodextrin in the combination for which ibuprofen solubility is maximum defines the stoichiometry of the complex.

The pH of each solution was measured using an Orion Model 701 pH meter with a combination electrode.

Results and Discussions

Heptakis (2,6-di-O-methyl)-β-cyclodextrin:

Table I below lists the compositions studied and the concentration and pH measurements of the resulting solutions for combinations of ibuprofen with heptakis(2,6-di-O-methyl)-β-cyclodextrin. Figure 1 of the attached drawing shows the relationship between mole ratio of the starting materials and both ibuprofen solution concentration and pH. From this figure we see that the concentration is a maximum and the pH a minimum at a mole ratio of 1:1, indicating a complex with one molecule each of heptakis (2,6-di-O-methyl)-β-cyclodextrin and of ibuprofen. The maximum concentration of the complex corresponds to about 53 mg of ibuprofen in 5 ml, nearly 180 times the saturation concentration of the drug alone. The pH, about one unit lower than that of a saturated solution of the ibuprofen, could result from the increased concentration of the acidic drug. The stoichiometry indicates that one molecule of ibuprofen is entrapped in the cavity of the heptakis (2,6-di-O-methyl)-β-cyclodextrin.

γ-Cyclodextrin:

Data for combinations of ibuprofen with γ-CD are listed in Table II below. Figure 2 of the attached drawing shows the relationship between mole ratio of the starting materials and both ibuprofen solution concentration and pH. From this figure we see that the concentration is a maximum at a mole ratio of 1:2, indicating a complex with two molecules of ibuprofen entrapped in the relatively large cavity of the γ-cyclodextrin. The maximum concentration of the complex corresponds to 0.69 mg of ibuprofen in 5 ml, about 2 times the saturation concentration of the drug alone. The pH of the 1:2 complex is slightly higher than that of the nearest other mixtures studied. At this time we have no explanation of this phenomenom.

α-Cyclodextrin:

Data for combinations of ibuprofen with a α-CD are listed in Table III below. Figure 3 of the attached drawings shows the relationship between mole ratio fo the starting materials and both ibuprofen solution concentration and pH. From this figure we see that the concentration is a maximum and the pH a minimum at a mole ratio of about 9:1. The large excess of α-CD implies that there is is no inclusion in the relatively small internal cavity, but, perhaps, a "chain-type" complex is formed. Nevertheless, a considerable increase in ibuprofen solubility, about 20-fold, is observed.

TABLE I

HEPTAKIS(2,6-di-O-METHYL)-β-CYCLODEXTRIN-IBUPROFEN COMPLEX

| Mole Ratio - Cyclo-dextrin: Ibuprofen | Initial Combination | | | | Solution | | |
|---|---|---|---|---|---|---|---|
| | Cyclodextrin | | Ibuprofen | | Ibuprofen Concentration | | |
| | mg | moles | mg | moles | mg/5ml | moles/5ml | pH |
| 1:0 | 634.0 | $4.76 \times 10^{-4}$ | 0 | 0 | 0 | 0 | 4.90 |
| 2:1 | 634.0 | $4.76 \times 10^{-4}$ | 50.0 | $2.42 \times 10^{-4}$ | 38.7 | $1.88 \times 10^{-4}$ | 3.59 |
| 3:2 | 570.0 | $4.28 \times 10^{-4}$ | 57.8 | $2.80 \times 10^{-4}$ | 38.4 | $1.86 \times 10^{-4}$ | 3.55 |
| 1:1 | 475.4 | $3.57 \times 10^{-4}$ | 72.3 | $3.51 \times 10^{-4}$ | 53.1 | $2.57 \times 10^{-4}$ | 3.54 |
| 1:3 | 238.4 | $1.79 \times 10^{-4}$ | 115.5 | $5.60 \times 10^{-4}$ | 27.5 | $1.33 \times 10^{-4}$ | 3.64 |
| 1:4 | 191.2 | $1.44 \times 10^{-4}$ | 116.1 | $5.63 \times 10^{-4}$ | 23.1 | $1.12 \times 10^{-4}$ | 3.69 |
| 0:1 | 0 | 0 | 130.0 | $6.30 \times 10^{-4}$ | 0.30 | $1.45 \times 10^{-6}$ | 4.56 |

TABLE II

γ-CYCLODEXTRIN - IBUPROFEN COMPLEX

| Initial Combination | | | | | Solution | | |
|---|---|---|---|---|---|---|---|
| Mole Ratio - Cyclo-dextrin: Ibuprofen | Cyclodextrin | | Ibuprofen | | Ibuprofen Concentration | | |
| | mg | moles | mg | moles | mg/5ml | moles/5ml | pH |
| 1:0 | 605.8 | $4.67 \times 10^{-4}$ | 0 | 0 | 0 | 0 | 5.59 |
| 2:1 | 608.1 | $4.67 \times 10^{-4}$ | 49.1 | $2.38 \times 10^{-4}$ | 0.37 | $1.77 \times 10^{-6}$ | 4.52 |
| 1:1 | 453.7 | $3.50 \times 10^{-4}$ | 72.1 | $3.50 \times 10^{-4}$ | 0.56 | $2.72 \times 10^{-6}$ | 4.39 |
| 1:2 | 300.5 | $2.32 \times 10^{-4}$ | 97.4 | $4.72 \times 10^{-4}$ | 0.69 | $3.35 \times 10^{-6}$ | 4.46 |
| 1:4 | 181.9 | $1.40 \times 10^{-4}$ | 115.4 | $5.59 \times 10^{-4}$ | 0.61 | $2.94 \times 10^{-6}$ | 4.32 |
| 1:9 | 90.4 | $7.00 \times 10^{-5}$ | 131.6 | $6.38 \times 10^{-4}$ | 0.46 | $2.18 \times 10^{-6}$ | 4.40 |
| 0:1 | 0 | 0 | 130.0 | $6.30 \times 10^{-4}$ | 0.30 | $1.45 \times 10^{-6}$ | 4.56 |

TABLE III

α-CYCLODEXTRIN - IBUPROFEN COMPLEX

| Initial Combination | | | | | Solution | | |
|---|---|---|---|---|---|---|---|
| Mole Ratio - Cyclo-dextrin: Ibuprofen | Cyclodextrin | | Ibuprofen | | Ibuprofen Concentration | | |
| | mg | moles | mg | moles | mg/5ml | moles/5ml | pH |
| 1:0 | 647.0 | $6.65 \times 10^{-4}$ | 0 | 0 | 0 | 0 | 6.30 |
| 19:1 | 647.0 | $6.65 \times 10^{-4}$ | 7.9 | $3.83 \times 10^{-5}$ | 6.25 | $3.03 \times 10^{-5}$ | 4.02 |
| 9:1 | 613.0 | $6.30 \times 10^{-4}$ | 14.3 | $6.93 \times 10^{-5}$ | 6.50 | $3.15 \times 10^{-5}$ | 3.91 |
| 17:3 | 578.8 | $5.95 \times 10^{-4}$ | 21.9 | $1.06 \times 10^{-4}$ | 6.25 | $3.03 \times 10^{-5}$ | 3.93 |
| 4:1 | 545.6 | $5.60 \times 10^{-4}$ | 29.0 | $1.41 \times 10^{-4}$ | 5.65 | $2.74 \times 10^{-5}$ | 3.97 |
| 1:1 | 340.4 | $3.49 \times 10^{-4}$ | 71.9 | $3.49 \times 10^{-4}$ | 3.66 | $1.77 \times 10^{-5}$ | 3.99 |
| 1:4 | 136.3 | $1.40 \times 10^{-4}$ | 115.9 | $5.58 \times 10^{-4}$ | 1.69 | $8.19 \times 10^{-6}$ | 4.28 |
| 0:1 | 0 | 0 | 130.3 | $6.30 \times 10^{-4}$ | 0.30 | $1.45 \times 10^{-6}$ | 4.56 |

## Claims

1. As a composition of matter an ibuprofen - cylodextrin complex said cyclodextrin being selected from the group consisting of α-cyclodextrin, a methylated-β-cyclodextrin and γ-cyclodextrin.
2. A composition according to Claim 1 wherein said cyclodextrin is α-cyclodextrin.
3. A composition according to Claim 2 wherein the mole ratio of α-cyclodextrin to ibuprofen in said complex is about 9:1.
4. A composition according to Claim 1 wherein the cyclodextrin is heptakis(2,6-di-O-methyl)-β-cyclodextrin.
5. A composition according to Claim 4 wherein the mole ratio of heptakis(2,6-di-O-methyl)-β-cyclodextrin to ibuprofen in said complex is about 1:1.
6. A composition according to Claim 1 wherein said cyclodextrin is γ-cyclodextrin.
7. A composition according to Claim 6 wherein the mole ratio of γ-cyclodextrin is ibuprofen is about 1:2.
8. A process for preparing an ibuprofen-cyclodextrin complex which comprises mixing ibuprofen and a cyclodextrin in a solution at elevated temperatures over a period of time to form said ibuprofen-cyclodextrin complex and recovering the same, said cyclodextrin being selected from the group consisting of α-cyclodextrin, a methylated-β-cyclodextrin and γ-cyclodextrin.
9. A process according to Claim 8 wherein said solution is an aqueous solution.
10. A process according to Claim 9 wherein said solution is mixed at a temperature of about 55°C overnight.
11. A process according to Claim 10 wherein said complex is recovered by cooling said mixture to a temperature of about 25°C, filtering said cooled solution to form a clear solution, and removing the liquid from said clear solution and collection said complex as a solid.
12. A process according to Claims 8, 9, 10, or 11 wherein said cyclodextrin is α-cyclodextrin.
13. A process according to Claim 12 wherein said α-cyclodextrin and said ibuprofen are present in said solution in the molar ratio of about 9:1.
14. A process according to Claims 8, 9, 10 or 11 wherein said cyclodextrin is a methylated-β-cyclodextrin.
15. A process according to Claims 8, 9, 10 or 11 wherein said cyclodextrin is a heptakis(2,6,-O-methyl)-β-cyclodextrin.
16. A process according to Claim 15 wherein said heptakis (2,6,-O-methyl)-β-cyclodextrin and ibuprofen are present in said solution in the molar range of about 1:1.
17. A process according to Claims 8, 9, 10 or 11 wherein said cyclodextrin is γ-cyclodextrin.
18. A process according to Claim 17 wherein said γ-cyclodextrin and ibuprofen are present in said solution in the molar range of about 1:2.
19. A therapeutic composition comprising a pharmaceutically acceptable carrier having incorporated therein a therapeutically effective amount of a complex as defined in any one of Claims 1 to 7.
20. A therapeutic composition comprising a pharmaceutically acceptable carrier having incorporated therein a therapeutically effective amount of a complex as defined in any one of Claims 1 to 7, said complex being present therein at an effective daily dose of from about 200 mg/day to about 9,000 mg/day.
21. A therapeutic composition in unit dosage form comprising a pharmaceutically acceptable carrier having incoporated therein a therapeutically effective amount of a complex as defined in any one of Claims 1 to 7, said complex being present in said composition in the range of 200 mg to about 3,000 mg per unit dose.
22. A complex as defined in any one of Claims 1 to 7 for use in a method of treatment of the human or animal body.
23. A complex according to Claim 22 for use in the treatment as an analgesic or anti-inflammatory agent.
24. Use of a complex according to any one of Claims 1 to 7 in a process for the preparation of a medicament which is an analgesic or anti-inflammatory agent.

Claims for the following contracting states: Es, Gr.

1. A process of preparing an ibuprofen - cyclodextrin complex by combining ibuprofen with said cyclodextrin selected from α-cyclodextrin, a methylated-β-cyclodextrin or γ-cyclodextrin.
2. A process according to Claim 1 which comprises mixing ibuprofen and the cyclodextrin in a solution at elevated temperatures over a period of time to form said ibuprofen-cyclodextrin complex and recovering the same, said cyclodextrin being selected from the group consisting of α-cyclodextrin, a methylated-β-cyclodextrin and γ-cyclodextrin.
3. A process according to Claim 2 wherein said solution is an aqueous solution.
4. A process according to either of Claims 2 and 3 wherein said solution is mixed at a temperature of about 55°C overnight.
5. A process according to Claim 4 wherein said complex is recovered by cooling said mixture to a temperature of about 25°C, filtering said cooled solution to form a clear solution, and removing the liquid

from said clear solution and collection said complex as a solid.

6. A process according to any one of Claims 1 to 5 wherein there is used as said cyclodextrin, α-cyclodextrin.

7. A process according to Claim 6 wherein the α-cyclodextrin and ibuprofen are combined in said complex in a mole ratio of about 9:1.

8. A process according to any one of Claims 1 to 5 wherein the cyclodextrin used is a methylated-β-cyclodextrin.

9. A process according to any one of Claims 1 to 5 wherein there is used as the cyclodextrin, heptakis(2,6-di-O-methyl)-β-cyclodextrin.

10. A process according to Claim 9 wherein the heptakis(2,6-di-O-methyl)-β-cyclodextrin and ibuprofen are combined in said complex in a ratio of about 1:1.

11. A process according to any one of Claims 1 to 5 wherein there is used as said cyclodextrin, γ-cyclodextrin.

12. A process according to Claim 1 wherein the γ-cyclodextrin and ibuprofen combined in a mole ratio of about 1:2.

13. A process according to any one of Claims 1 to 12 wherein the resulting complex is formed into therapeutic composition by combining a pharmaceutically acceptable carrier with a therapeutically effective amount of the complex.

14. A process according to any one of Claims 1 to 12 wherein the resulting complex is formed into therapeutic composition in unit dosage form by combining a pharmaceutically acceptable carrier with a therapeutically effective amount of the complex said complex being present in said final composition in the range of 200 mg to about 9,000 mg per unit dose.

15. A process according to Claim 14 wherein the complex is added in amounts of from 200 mg to 3,000 mg per unit dose.

16. Use of a complex of ibuprofen with a cyclodextrin chosen from α-cyclodextrin methylated, -β-cyclodextrin or a γ-cyclodextrin in a process for preparing a medicament for use as an analgesic or anti-inflammatory agent.

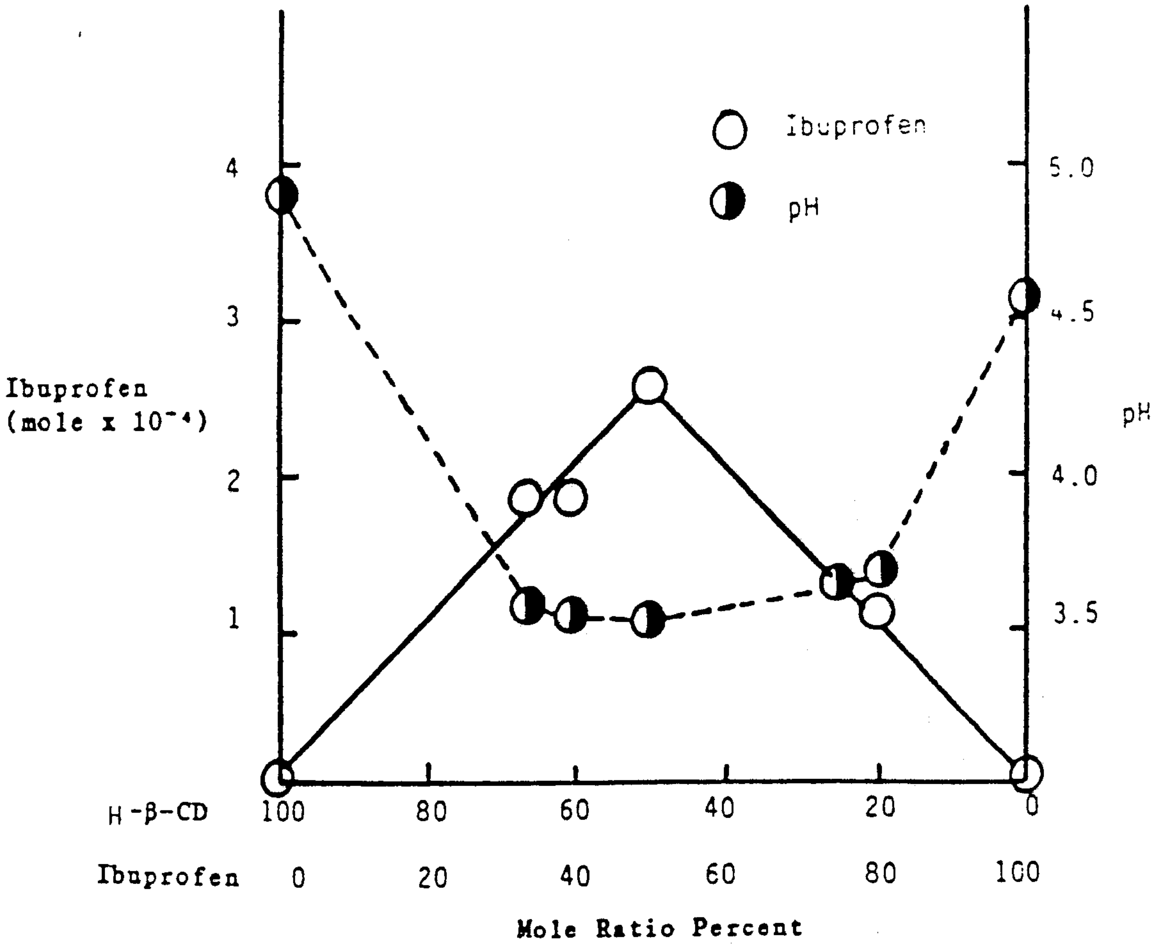
Ibuprofen
pH
Ibuprofen (mole x 10⁻⁴)
4
3
2
1
5.0
4.5
4.0
3.5
pH
H-β-CD
100
80
60
40
20
0
Ibuprofen
0
20
40
60
80
100
Mole Ratio Percent

FIG. 1

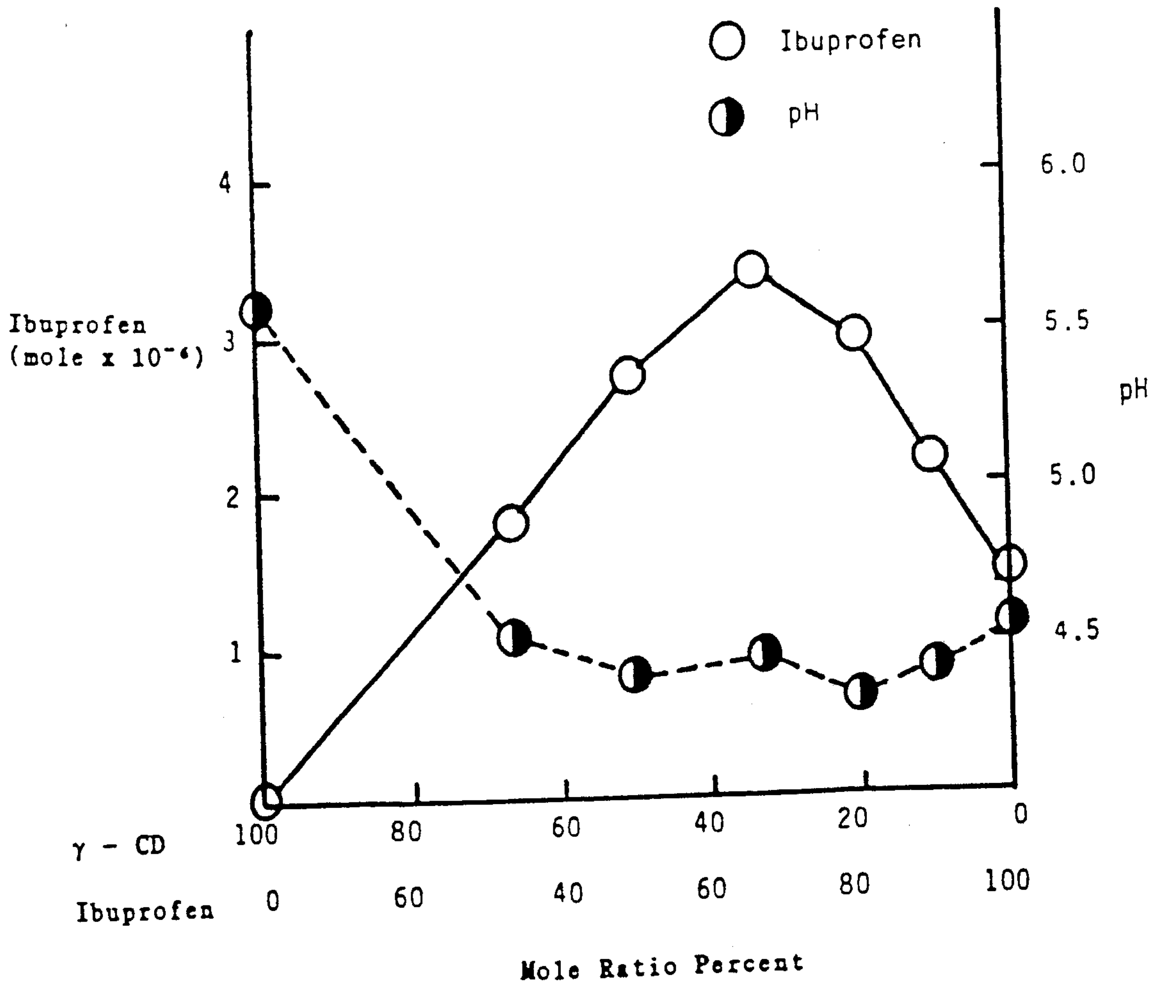

Ibuprofen
pH
4
3
2
1
Ibuprofen
(mole x 10⁻⁶)
6.0
5.5
5.0
4.5
pH
γ - CD
100
80
60
40
20
0
Ibuprofen
0
60
40
60
80
100
Mole Ratio Percent


FIG. 2

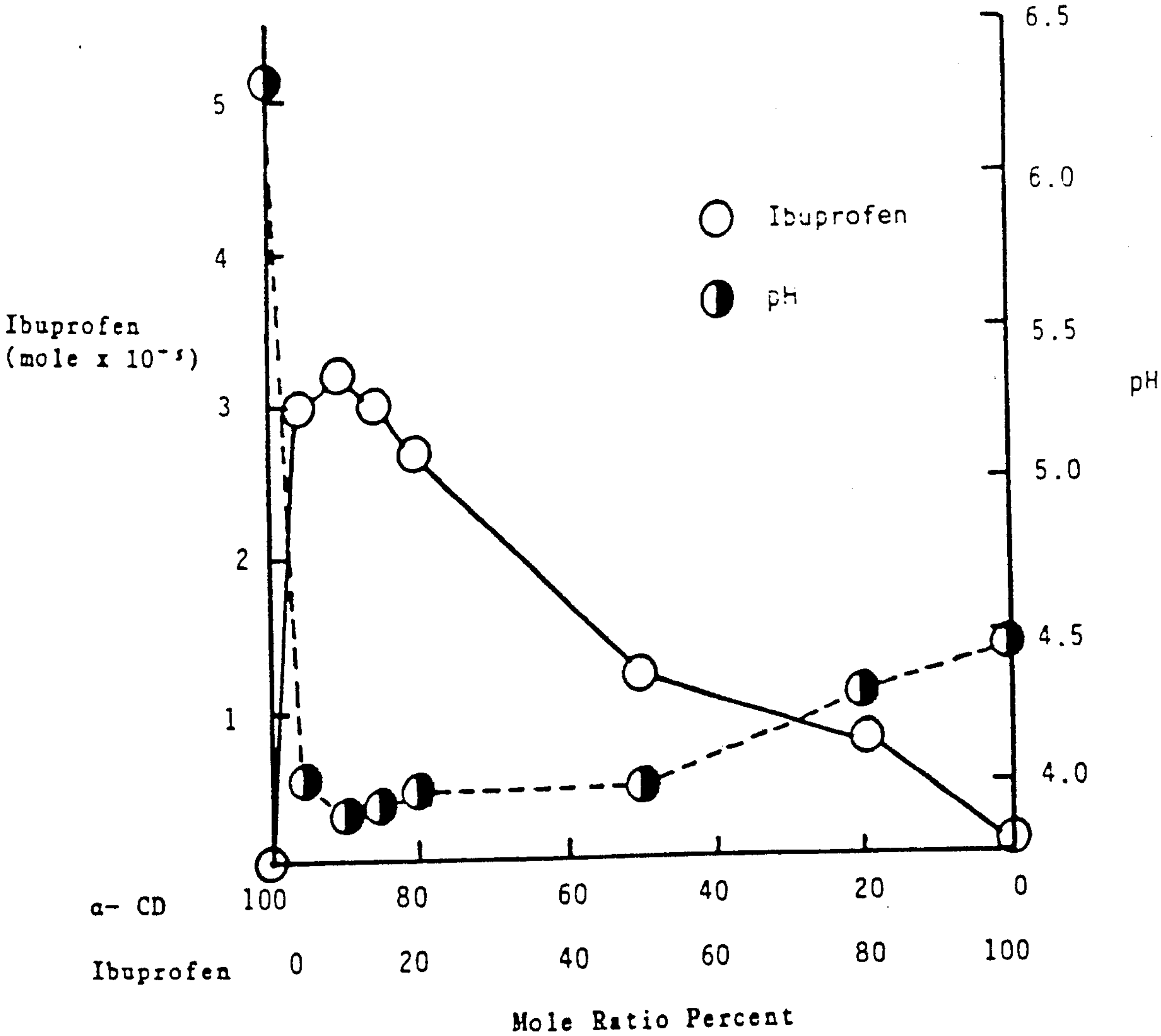

6.5
6.0
5.5
5.0
4.5
4.0
5
4
3
2
1
Ibuprofen
pH
Ibuprofen
(mole x 10⁻⁵)
pH
α- CD
100
80
60
40
20
0
Ibuprofen
0
20
40
60
80
100
Mole Ratio Percent


FiG. 3

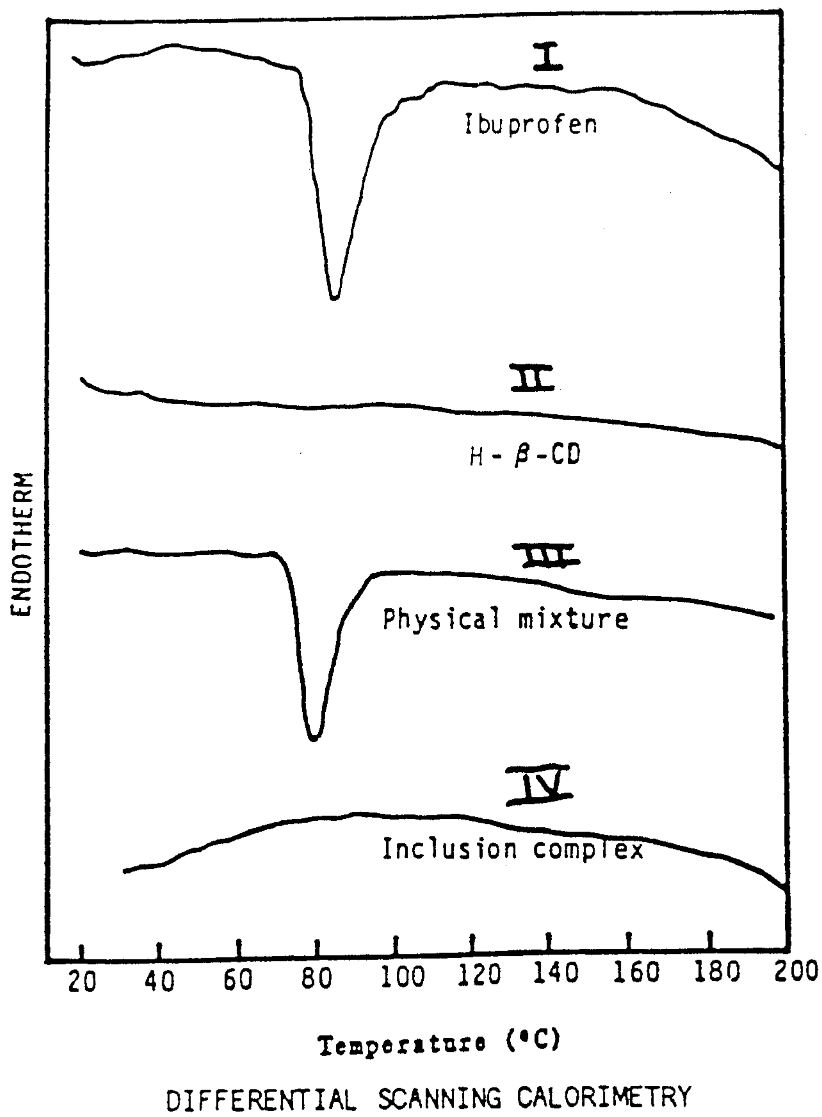
I
Ibuprofen
II
H-β-CD
III
Physical mixture
IV
Inclusion complex
ENDOTHERM
20
40
60
80
100
120
140
160
180
200
Temperature (°C)
DIFFERENTIAL SCANNING CALORIMETRY

FIG. 4

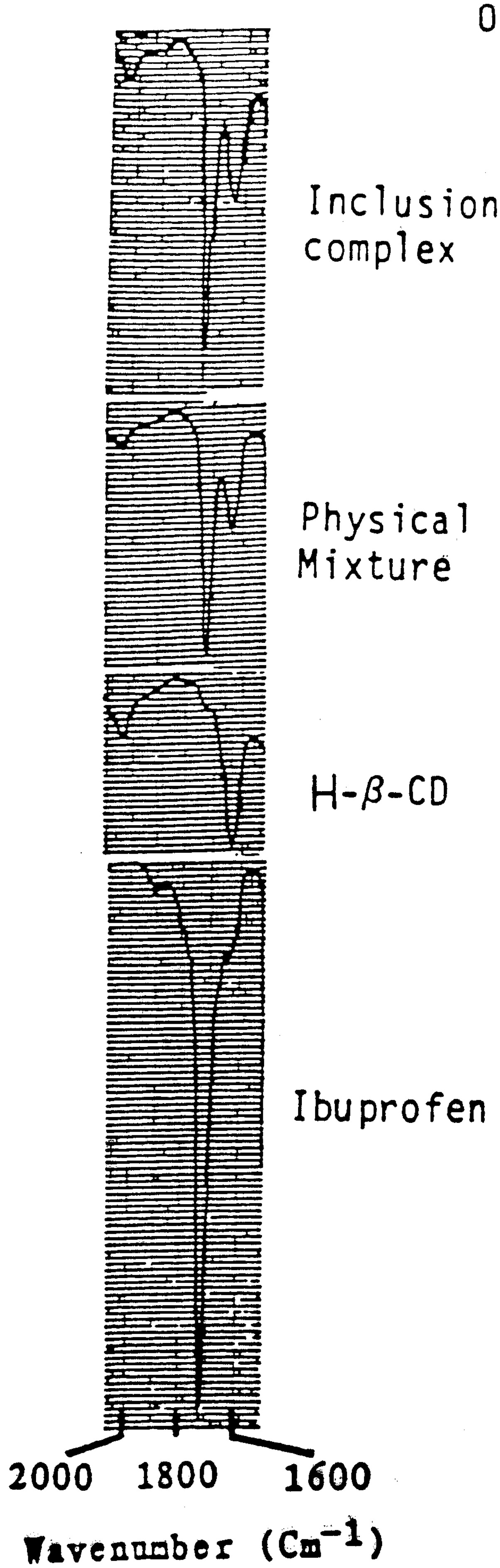


INFRARED SPECTRA

FIG.5